(19) Europäisches Patentamt
European Patent Office
Office européen des brevets

(11) Publication number : **0 261 239 B1**

# (12) EUROPEAN PATENT SPECIFICATION

(45) Date of publication of patent specification : **15.07.92 Bulletin 92/29**

(51) Int. Cl.$^5$ : **A61K 37/43,** C07K 13/00

(21) Application number : **87903070.8**

(22) Date of filing : **03.04.87**

(86) International application number : **PCT/US87/00790**

(87) International publication number : **WO 87/05810 08.10.87 Gazette 87/22**

## (54) FSH-RELEASING PEPTIDE.

(30) Priority : **04.04.86 US 848363**
**07.04.86 US 849108**

(43) Date of publication of application : **30.03.88 Bulletin 88/13**

(45) Publication of the grant of the patent : **15.07.92 Bulletin 92/29**

(84) Designated Contracting States : **BE CH DE FR GB IT LI NL SE**

(56) References cited :
**EP-A- 0 222 491**
**NATURE, vol. 321, 19th June 1986; W. VALE et al., pp. 776-779**
**NATURE, vol. 318, 19th/26th December 1985; A.J. MASON et al., pp. 659-663**
**NATURE, vol. 321, 19th June 1986; N. LING et al., pp. 779-782**
**PROCEEDINGS OF THE NATIONAL ACADEMY OF SCIENCES, vol. 82, November 1985, pp. 7217-7221**
**BIOCHEMICAL & BIOPHYSICAL RESEARCH COMMUNICATIONS, vol. 133, no. 1, 27th November 1985, pp. 120-127**
**BRAIN RESEARCH BULLETIN, vol. 10, 22nd March 1983, pp. 623-629**

(73) Proprietor : **THE SALK INSTITUTE FOR BIOLOGICAL STUDIES**
**10010 North Torrey Pines Road**
**La Jolla California 92037 (US)**

(72) Inventor : **VALE, Wylie Walker Jr.**
**1643 Valdez**
**La Jolla, CA 92037 (US)**
Inventor : **RIVIER, Jean Edouard Frederic**
**9674 Blackgold Road**
**La Jolla, CA 92037 (US)**
Inventor : **SPIESS, Joachim**
**271 Cerro Street**
**Encinitas, CA 92024 (US)**
Inventor : **LING, Nicholas Chai-Kwan**
**5324 Bloch Street**
**San Diego, CA 92122 (US)**
Inventor : **YING, Shao-Yao**
**3305 Willard Street**
**San Diego, CA 92122 (US)**
Inventor : **ESCH, Frederick Stephen**
**2929 Fire Mountain Drive 63**
**Oceanside, CA 92057 (US)**
Inventor : **GUILLEMIN, Roger Charles Louis**
**7316 Encelia Drive**
**La Jolla, CA 92037 (US)**

(74) Representative : **Lawrence, Malcolm Graham et al**
**Hepworth, Lawrence & Bryer 2nd Floor, Gate House South Westgate Road**
**Harlow Essex CM20 1JN (GB)**

EP 0 261 239 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to peptides which affect the release of hormones from the pituitary and more particularly to a protein which promotes the release of follicle-stimulating hormone from the anterior lobe of the pituitary gland.

## BACKGROUND OF THE INVENTION

There has been considerable interest in hormones which affect the release of hormones from the anterior lobe of the pituitary gland, in particular luteinizing hormone (LH) and follicle-stimulating hormone (FSH). A variety of hypophysiotropic peptides or proteins have been reported to be present in mammalian gonads. Gonadotropin releasing hormone (GnRH), a decapeptide, which is also known as luteinizing hormone release factor (LRF) is a protein found to stimulate the release of both LH and FSH. Proteins termed "inhibins", which selectively suppress the secretion of FSH but, under most circumstances, do not suppress the release of LH, have been recently isolated and characterized from gonadal fluids of several species, Robertson et al., Biochem. Biophys. Res. Commun. 126, 220-226 (1985); Miyamoto et al., Biochem. Biophys. Res. Commun. 129, 396-503 (1985); Rivier et al., Biochem. Biophys. Res. Commun. 133, 120-127 (1985); Ling et al., P.N.A.S. 82, 7217-7221 (1985). Mason et al., Nature, 318, 659-663 (1985) disclosed the complete amino acid sequences of porcine inhibin $\alpha$ subunit and two distinct $\beta$ subunits, $\beta_A$ and $\beta_B$. Gonadotropin-releasing peptides have also been described in the gonads which have generally been shown to have activity in radioreceptor assays for GnRH but to exhibit different chromatographic and immunologic characteristics from that of GnRH, Igarashi et al. Endocrinology 74: 446-452 (1964); Igarashi et al., In Psychoncuroendocrinology, ed. by Hatotani et al., pp. 178-186 (1973); Lundanes et al., Biochem. Biophys. Res. Commun. 94: 827-836 (1980) and Samson et al., Peptides 1:97-102 (1980).

Described herein is a protein which is found in mammalian follicular fluid and which has action opposite that of inhibin in that it stimulates the release of FSH from the pituitary but does not stimulate the release of LH. This peptide is designated "FSH-releasing peptide"(FRP) and has been purified to substantial homogeneity.

## SUMMARY OF THE INVENTION

In accordance with the present invention, a protein having a molecular weight of about 28,000 daltons (28 kD) and having FSH-releasing activity has been successfully isolated to substantial homogeneity from porcine follicular fluid(pFF). The 28 kD protein exhibits FRP activity in that it specifically stimulates the secretion and synthesis of FSH by cultured rat anterior pituitary cells but does not stimulate release of LH or other known pituitary hormones.

The protein has a molecular weight of about 28 kD as determined by sodium dodecyl sulfate-polyacrylamide gel electrophoresis (SDS-PAGE); under reducing conditions a major band with apparent molecular weight of 14-16 kD is observed which suggests that FRP is a dimer. Indications are that FRP is a dimer of inhibin $\beta_A$ chains and that such $\beta_A$ inhibin chain dimers, and perhaps monomers which are unlinked to inhibin alpha chains, are functional antagonists of inhibin activity and may be administered to a mammalian animal to counteract endogenous inhibin and therefore act in a direction opposite to that of inhibin.

Purification of 28 kD porcine FRP to a purity of at least about 90% by weight of total protein in the fraction was achieved through a combination of protein separation procedures including salt precipitation, ultrafiltration, preparatory high-performance liquid chromatography (HPLC), gel filtration, cation exchange-fast protein liquid chromatography (FPLC), semi-preparatory HPLC, gel permeation-FPLC and analytical HPLC using a Vydac $C_8$ stationary phase and a TFA/$CH_3CN$ (trifluoroacetic acid/acetonitrile) gradient mobile phase.

## BRIEF DESCRIPTION OF THE DRAWINGS

Figure 1 is an elution profile of FRP on cation exchange chromatography. The broken line indicates the NaCl gradient concentration from 0 to 1.0 M during the course of the run. The shaded area indicates FSH activity per well determined by bioassay, as described herein, the activity being expressed as the ng of FSH released per well at 72 hours. The solid line represents the concentration of total protein eluted as monitored at 280 nm using an ultraviolet detector.

Figure 2 is an elution profile of FRP during the final analytical reverse-phase, high-performance liquid chromatography (RP-HPLC) fractionation.

## DETAILED DESCRIPTION OF THE PREFERRED EMBODIMENTS

In accordance with the present invention a protein, designated FRP, is discovered in mammalian follicular fluid which stimulates the secretion of FSH from cultured anterior pituitary cells but does not appear to stimulate the release of LH or any other known pituitary hormone. In this respect, the action of FRP is opposite to the action of inhibin, which inhibits basal secretion of FSH from cultured anterior pituitary cells but does not inhibit basal release of LH. The action of FRP is not blocked by known GnRH antagonists which interact with GnRH receptors, indicating that the biological activity of FRP is not through interaction with GnRH receptors. The protein can be isolated in a multi-step procedure to substantial homogeneity, i.e., greater than about 75% by weight of total endogenous protein.

Physically, the major component has a molecular weight of about 28,000 daltons as determined by SDS-PAGE. Under reducing conditions, a predominant zone of approximately 14,000 daltons is seen, indicating that the protein is a dimer. At the end of a multi-step purification procedure, the protein is further characterized by means of analytical RP-HPLC using a stationary phase of Vydac $C_8$, 5 µm, in a 0.46 x 25 cm. column, and a gradient of buffer A, which is 0.1% aqueous trifluoroacetic acid (TFA), and buffer B, which is 0.1% TFA plus a mixture of water and acetonitrile (20:80). Unless otherwise noted, all ratios and percents are by volume. As shown in Fig. 2, the FRP material is loaded with an initial flow of 20% buffer B and a flow rate of 1.2 ml/min. After loading, the column is eluted at a flow rate of 0.7 ml/min. and a temperature of 40°C. so as to achieve a gradient which results in a 45% buffer B solution in 30 minutes. The protein elutes from the column, and at about 39 minutes after the starting the gradient, a single, sharp band is collected, which is tested for biological activity and potency and found to be FRP. It is then characterized by Edman degradation.

FRP was initially isolated from porcine follicular fluid (pFF), generally contemporaneously with isolation of inhibin, Rivier et al., Biochem. Biophys. Res. Commun. (1985) supra. Some chromatographic elution fractions of pFF were found to inhibit FSH secretion from anterior pituitary cells, i.e., they exhibited inhibin activity; whereas other fractions were found to stimulate the secretion of FSH by the cultured cells, and the active material in the FSH-stimulating fractions was designated FRP. Both FRP and inhibin fractions were further processed to isolate the active proteins, purified FRP being the subject of the present invention.

Briefly, the initial purification of FRP to substantial homogeneity proceeded as follows.

A salt precipitation step, an ultrafiltration step and a preparative HPLC step were part of the procedure by which porcine inhibin was purified from pFF. Dialyzed supernatant from a 50% ammonium sulfate precipitation of about 6 liters of pFF was ultrafiltered to remove protein having molecular weights below about 10 kD and then subjected to preparative HPLC using a Vydac $C_4$ stationary phase and a TEAP, pH 6.5 buffer, propanol gradient mobile phase as described earlier in Rivier et al., Biochem. Biophys. Res. Commun. (1985) Supra. FSH-releasing activity was observed in the early eluting, retarded fractions, and was thereby separated from the two major FSH-release-inhibiting (inhibin) zones, which were either not retarded or were eluted later.

Throughout the purification procedure, FRP activity in elution fractions was determined by a bioassay. The bioassay for FRP is based upon the ability of FRP to stimulate FSH secretion by cultured anterior pituitary cells as described in Vale et al. Endocrinology, 91, 562-572 (1972). Using this assay, half-maximal effective dosages ($EC_{50}$) are determined for FRP and FRP-containing fractions. The half-maximal effective dose is the concentration of protein that increases the basal secretion of FSH to one-half the plateau level of increased FSH secretion, i.e., where additional FRP does not further stimulate FSH secretion. The $EC_{50}$'s herein are based upon a plating of 5 x $10^5$ pituitary cells incubated for 2.5 hours at 37°C in 60 x 15 mm Falcon culture dishes containing 3 ml of Dulbecco modified Eagle's medium (DMEM) plus 100 µl of protein-containing solution.

The zones obtained from the preparative RP-HPLC which exhibited FSH-releasing activity in bioassay were pooled, lyophilized and redissolved in a strongly dissociating buffer which contained 6 M guanidine·HCl. The solution was then gel filtrated by application to a Sepharose CL-6B column and FSH-releasing activity was eluted with the same buffer with over a $K_{AV}$ range of from 0.54 to 0.71. It was noted at this point that FRP behaved on gel filtration similarly to the 32K form of inhibin, which had fortunately been separated earlier by the preparative HPLC.

The FSH-releasing fractions were pooled and dialyzed and then subjected to cation exchange chromatography on FPLC using a Mono-S (Mono-S is a trade mark) stationary phase equilibrated in a solution of 4 M urea, 50 mM sodium acetate and eluted by a salt gradient of from 0 to 1 M NaCl in the solution. Two regions of FSH-releasing activity were detected, FRP-I and FRP-II (Fig. 1).

The latter eluting FRP-II fraction had higher specific activity and was further purified by semi-preparative RP-HPLC using a Vydac $C_8$ (Vidac $C_8$ is a trade mark) column and a NaCl gradient in TFA/$CH_3CN$ solution. Active fractions from the semi-prep RP-HPLC were further purified by gel permeation on a FPLC Superose 12B (Superose is a trade mark) column eluted with 6 M guanidine·HCl. Finally, FRP was isolated by two steps of analytical RP-HPLC using a Vydac $C_8$ column eluted with a $CH_3CN$ gradient in TFA/$CH_3CN$ solution. The FRP eluting from the final analytical HPLC produced a single peak of activity as seen in Figure 2. On SDS-PAGE under reducing conditions, the protein in the pooled peak fractions ran as a single band, corresponding to a molecular weight of approximately 14 kD.

Highly purified FRP stimulates FSH secretion by cultured anterior pituitary cells in a dose-related fashion, exhibiting a half-maximal effective dose ($EC_{50}$) of about 25 pM, assuming a molecular weight of 28 kD. FRP does not stimulate the secretion of pituitary hormones LH, prolactin (Prl), growth hormone (GH) or adrenocorticotrophin (ACTH) and, in fact, slightly suppresses basal release of GH and ACTH.

FRP is distinguished in activity from GnRH in several ways. Although FRP is a potent releaser of FSH, it has no effect on the secretion of LH, whereas GnRH releases both gonadotropins. GnRH acts immediately to stimulate gonadotropin secretion, whereas the onset of action of FRP is delayed by from 4 to 24 hours and is not maximally effective until more than 24 hours. However, pituitary cells treated with maximal concentrations of FRP for 72 hours secrete as much FSH during that period as those exposed to plateau levels of GnRH.

GnRH receptor antagonists, which in vitro completely prevent the release of FSH and LH that is attributable to GnRH, have no effect on the secretion of FSH mediated by FRP. These results strongly indicate that FRP acts independently of the GnRH receptor.

Whereas prolonged exposure of pituitary cells to GnRH results in considerable depletion of cellular FSH stores, FRP, which mediates the release of similar amounts of FSH into the medium, actually increases the amounts of stored FSH within the cells. FRP, therefore, appears to have greater effect on the biosynthesis of FSH than does GnRH.

Purified porcine inhibin lowers baseline production of FSH and therefore obscures the effects of modest concentrations of FRP. Inhibin and FRP are functionally competitive but each acts independently, i.e., in absence of the addition of the other. The existence of gonadal FRP may have been overlooked previously because of the presence of inhibin whose 32 kD form does not separate from FRP by gel permeation chromatographic methods.

There are strong indications from sequencing analysis that the 28 kD FRP protein is a homodimer of inhibin $\beta_A$ subunits in which cysteine residues of the chains are interconnected through disulfide bonds. Inhibin $\beta_B$ homodimers, $\beta_a\beta_B$ heterodimers and $\beta_A$ and $\beta_B$ monomers might all act as functional antagonists of inhibin, which is itself a dimer of an $\alpha$-chain and a $\beta$-chain. Accordingly, the use of substantially purified inhibin $\beta$ chains, in monomeric or dimeric form, might be useful to selectively stimulate FSH secretion.

Although the invention is described herein primarily in terms of porcine FRP, and more specifically the dimeric form of inhibin $\beta_A$ chains, there is very strong homology between various vertebrate inhibin $\beta$ chains, particularly mammalian inhibin $\beta$ chains, e.g., porcine, human, ovine, bovine, etc. Accordingly, such vertebrate $\beta$ chains, in general, are believed to have biological cross-reactivity between vertebrate species in stimulating FSH secretion.

The isolation of 28 kD porcine FRP to substantial homogeneity is now described in greater detail by way of specific example.

## EXAMPLE

Bioassay of FSH-releasing activity, indicating the presence of the FRP in elution fractions throughout the purification, was performed as follows. Rat anterior pituitary glands were enzymatically dissociated anterior pituitary glands were enzymatically dissociated and the cells plated in a monolayer of cells provided in individual wells in B-PJ medium in 2% FBS. After washing the cells 3 times with the same medium, aliquots of eluants were added and allowed to remain on the cells for 48-72 hours, at which time fluids were removed from the cells and assayed for FSH using a radioimmuno assay kit provided by the National Hormone and Pituitary Program of NIADDK (The National Institute of Arthritis, Diabetes, Digestive and Kidney Disease). For testing elution fractions of RP-HPLC columns, aliquots for assays (0.1 - 1.0% of total fraction volume, but never less than 5 µl) were measured with micropipettes and plastic tips and were transferred into polypropylene tubes containing bovine serum albumin (10 µl of 10 mg/ml) and dried in a Savant rotary evaporator. For testing elution fractions of gel permeation and ion exchange columns where desalting was necessary prior to assay, small aliquots were removed and transferred into glass tubes containing 0.5 ml of 10 mM Hepes plus 0.1% BSA, pH 7.5. Squares of dialysis tubing with MW cutoff of ca. 1000 were secured over the tops with rubber bands. The tubes were inverted and dialyzed against 10 mM Hepes, pH 7.5. The retentates were then dried using a Savant rotary evaporator. All fractions were resuspended in the cell culture assay medium.

The starting material for the procedure, porcine follicular fluid(6050 mls), was supplied frozen by the Contraceptive Development Branch of NICHD (The National Institute of Child Health and Human Development). The material was thawed and centrifuged at 700 x g for 5 minutes in order to separate cell debris.

The crude supernatant was initially purified by ammonium sulfate salt precipitation. To the crude supernatant was added dropwise over 2-3 hours an equal volume of Schwartz/Mann ultrapure 100% saturated ammonium sulfate adjusted to pH 7.8 with ammonium hydroxide. The mixture was centrifuged at 8200 x g for 30 minutes, and the supernatant and precipitate were separated. The supernatant was retained.

The supernatant was ultrafiltered and concentrated using a Millipore Pellicon Cassette system with about 0.465 $m^2$ (5 sq. ft.) of filter of 10,000 MW cutoff at a rate of 300 ml/min. using a peristaltic pump with back pressure applied until filtration rate was 20 ml/min. The total retentate was washed several times with 10 mM Hepes and 0.05% dimethyl sulfide, pH 7. The final retentate(6,050 ml-eq in 2,240 ml.) was collected. The protein concentration of the retentate was 10 mg/ml-eq. The term "ml-eq" is used to indicate a given amount of biologically active protein material which is derived from 1 ml of pFF.

The concentrated retentate was next purified by preparative RP-HPLC. In each run, 500 ml-eq of protein or less were processed on a Waters Prep 500A using Vydac end-capped $C_4$ silicas, 15-20 $\mu$M particle size in a 5 x 30 cm cartridge. The column temperature was thermostated at 60°C. Buffer A was 0.1% $H_3PO_4$, 0.28% TEA (triethylene amine), pH 6.5; buffer B was 60% n-propanol in buffer A. Columns were loaded at 20% buffer B; the gradient used was 20% buffer B to 65% buffer B in 45 min. Flow rate was 75 mls/min. FSH-releasing activity eluted between retention volumes (RV) 825 and 1350 mls from the start of the gradient. Active zones from preparative RP-HPLC were pooled and lyophilized. The protein concentration was 700 $\mu$g/ml-eq; and on bioassay, the $EC_{50}$ was 2.8 $\mu$g/ml.

The pooled fractions from the preparative RP-HPLC were further purified by gel permeation FPLC on a 10 x 120(8600 ml.) Sepharose (Sepharose is a trade mark) CL-6B column. The pooled fraction was brought up to 6 M in guanidine·HCl, 0.1 M ammonium acetate, 0.05% dimethyl sulfide, pH 4.75. The eluant was 0.22 $\mu$m filtered and degassed before use. The flow rate was 180 mls/hr. FSH-releasing activity eluted between $K_{AV}$ 0.54 and 0.71. The pooled retentate of active fractions had a protein concentration of 130 $\mu$g/ml-eq and an $EC_{50}$ of 730 ng/ml.

The active fractions from gel permeation chromatography were pooled and dialyzed against Milli Q $H_2O$ plus 0.01% dimethyl sulfide using tubing with a MW cutoff of about 1000, and the fractions were lyophilized. The pooled retentate from the gel permeation was then further purified by cation exchange FPLC. Cation exchange was conducted using a Pharmacia FPLC system equipped with a Mono S®HR 16/10 column, $V_t$=20 ml. The mobile phase was a gradient of buffer A: 50 mM sodium formate, 4 M urea, 1 mM 3-[(Cholamidopropyl)dimethylammonio]-1-propanesulfonate (CHAPS) in Milli Q $H_2O$, pH 4.3, and buffer B: 1M -NaCl in buffer A. Buffers were 0.22 $\mu$m filtered and degassed before use. The lyophilized retentate was dissolved in buffer A and processed in three equal batches. Flow rate was 8 mls. per minute, and the gradient was as shown in Figure 1. In particular, for 9 min. the eluant was 100% buffer A; at 9 min. the buffer was abruptly changed to 15% buffer B and held thereat over a period from 32 to 36 min., the gradient was linearly increased from 15% buffer B to 50% buffer B; and in a period from 36 to 50 min., the buffer was linearly changed from 50% buffer B to 100% buffer B and the column was eluted at 100% buffer B thereafter.

Two biologically active zones of FRP, relative to baseline FSH-secretion, resulted from the cation exchange FPLC chromatography. The zone eluting from about 0.34 - 0.43 M NaCl is designated FRP-I. The later zone which eluted from about 0.43 - 0.60 M NaCl is designated FRP-II, and was the more active fraction. Pooled FRP-II fractions had a protein concentration of 8 $\mu$g/ml-eq and an $EC_{50}$ of 96 ng/ml.

Each of the pooled FRP-I and FRP-II eluates was further purified and desalted by semi-preparative RP-HPLC, using a Beckman HPLC and 1 x 30 Vydac columns packed with end-capped $C_8$ silicas, 5 $\mu$m particle size. The eluant was a gradient of buffer A: 0.1% TFA and buffer B: 80% $CH_3CN$ in 0.1% TFA. For FRP-I, columns were thermostated at 40°C and run at 2.5 ml/min. isocratically at 25% buffer B for 17 min., followed by a linear gradient to 55% buffer B in 30 min. Activity eluted between retention volumes 60 and 65 from the start of the gradient. The protein concentration of the FRP-II was 700 ng/ml-eq and the $EC_{50}$ on bioassay was 25 ng/ml.

Active zones from semi-preparative RP-HPLC of FRP-I and FRP-II were separately processed by gel permeation on the Pharmacia FPLC system using tandem Superose (Superose is trade mark) 12B columns, 10 $\mu$m, 10 x 300 mm each. Column eluant was 6 M guanidine·HCl, 0.1 M ammonium acetate, 0.05% dimethyl sulfide, pH 4.75 in Milli Q $H_2O$. The eluant was 0.22 $\mu$m filtered and degassed before use. Flow rate was 4 ml/min. In each case, activity was eluted between $K_{AV}$ 0.26 and 0.31. Pooled FRP-II fractions had a protein concentration of 300 ng/ml-eq and an $EC_{50}$ of 5 ng/ml.

The pooled active zones of FRP-II recovered from the FPLC gel permeation were processed by analytical RP-HPLC using a Vydac $C_8$, 5 $\mu$m, 0.46 x 25 cm column using the Beckman system, thermostated at 40°C. Buffer A was 0.1% TFA, buffer B was 0.1% TFA and a mixture of water and acetonitrile (20:80). Approximately 1,000 ml-eq were loaded at a time, starting initially at 20% buffer B and a loading flow rate of 1.2 ml/min. Injection volume was 4.3 mls in five equal injections. The gradient was run to 45% buffer B in 60 minutes after loading with a flow rate of 0.7 ml/min. Active fractions were pooled, and this step was repeated with the gradient being reduced to 30 minutes. The pooled fractions from this second step eluted as a single peak about 39 minutes after the gradient was begun, as seen in Figure 2. Protein concentration was 20 ng/ml-eq and $EC_{50}$ was 1 ng/ml. Thus about 100 $\mu$g of substantially homogeneous FRP protein is recovered from 390 gm. of total protein in the starting 6050 ml of pFF.

An aliquot of the highly purified FRP was subjected to Edman degradation. The first 32 residues of N-terminal sequence of the intact FRP were found to be identical to the N-terminal sequence of the porcine inhibin $\beta_A$-chain, i.e., Inhibin $\beta_A$ (1-32) or residues 309-340 (based on numbering in the pre pro form, Mason et al. supra.). Another aliquot of the FRP was reduced, S-carboxymethylated and subjected to HPLC. The major peak from HPLC was digested by clostripain, and the fragments, following HPLC purification, were subjected to Edman degradation. The sequences of certain fragments were found to be identical to residues 75-85, 88-102 and 103-116 of porcine inhibin $\beta_A$. This purified FRP exhibits a major band at 28 kD on SDS-PAGE, which following reduction converts to a major zone of 14-16 kD. These results are consistent with the conclusion that FRP-II is a homodimer consisting of two inhibin $\beta_A$ chains linked by disulfide bonds.

FRP is a homodimer of two subunits of 116 residues each of the following formula, interconnected by disulfide bonding: H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH. It is also possible to provide FSH-releasing biological activity by providing a synthetic dimer of two subunits of inhibin $\beta_B$, each of which has the formula:
H-Gly-Leu-Glu-Cys-Asp-Gly-Arg-Thr-Asn-Leu-Cys-Cys-Arg-Gln-Gln-Phe-Phe-Ile-Asp-Phe-Arg-Leu-Ile-Gly-Trp-Ser-Asp-Trp-Ile-Ile-Ala-Pro-Thr-Gly-Tyr-Tyr-Gly-Asn-Tyr-Cys-Glu-Gly-Ser-Cys-Pro-Ala-Tyr-Leu-Ala-Gly-Val-Pro-Gly-Ser-Ala-Ser-Ser-Phe-His-Thr-Ala-Val-Val-Asn-Gln-Tyr-Arg-Met-Arg-Gly-Leu-Asn-Pro-Gly-Thr-Val-Asn-Ser-Cys-Cys-Ile-Pro-Thr-Lys-Leu-Ser-Thr-Met-Ser-Met-Leu-Tyr-Phe-Asp-Asp-Glu-Tyr-Asn-Ile-Val-Lys-Arg-Asp-Val-Pro-Asn-Met-Ile-Val-Glu-Glu-Cys-Gly-Cys-Ala-OH. A heterodimer of inhibin $\beta_A$ and inhibin $\beta_B$ also has FSH-releasing biological activity. All of the above may be prepared synthetically, and biologically active fragments of either or both of the subunits may be used. For example, the C-terminus of one or both of the subunits may be shortened by removal of from 1 to 15 residues in a continuous sequence.

The 28 kD FRP protein is useful for regulating gonadotropin secretion and thus fertility and or sex hormone production of both male and female mammalians, particularly humans. Although the FRP isolated is of porcine origin, it is believed to be also biologically active throughout the genus of mammals, as have been the other neuro-regulatory hormones previously isolated, characterized and thereafter synthetically reproduced. It is conceivable that gonadal FRP has a wider distribution that might include the hypothalamus, and it is also possible that FRP plays important autocrine, paracrine and/or hormonal roles in the regulation of gonadal and perhaps other functions. The high potency of FRP on pituitary cells to modulate hormone secretion supports the regulatory potential of this protein.

Substantially pure 26 kD FRP or biologically active portions thereof or the nontoxic salts thereof, combined with a pharmaceutically acceptable carrier to form a pharmaceutical composition, may be administered to mammals, including humans, either intravenously, subcutaneously, percutaneously, intramuscularly or orally for control of fertility, gonadotropin secretion or sex hormone production. In particular, FRP holds promise for increasing male and female fertility both in man and other mammals.

Such peptides are often administered in the form of pharmaceutically acceptable nontoxic salts, such as acid addition salts or metal complexes, e.g., with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder, such as polylactide/glycolides, tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

FRP should be administered under the guidance of a physician, and pharmaceutical compositions will usually contain an effective amount of the peptide in conjunction with a conventional, pharmaceutically-acceptable carrier. The dosage will vary depending upon the specific purpose for which the protein is being administered, and daily dosage levels in the range of about 0.1 to about 2 milligrams per Kg. of body weight may be used when the protein is administered on a regular basis for fertility regulation. It appears that FRP will also be useful in treating infertility caused by low FSH production. Radioimmunoassays specific for FRP would be helpful in the diagnosis of fertility disorders in males and females.

Although the method of purification of FRP has been described primarily in terms of isolation from pFF, inhibin can be similarly purified from other crude extracts, including homan follicular fluid.

FRP monomers can be synthesized by recombinant DNA techniques in suitable cloning vectors. In certain suitable vector systems, dimers are completely synthesized. In other cases, the monomers are expressed by the vector systems and are then dimerized in vitro. Alternatively, it is conceivable that fragments of FRP small enough to be synthesized by solid-phase methods could be biologically active. The multi-step purification procedure, described herein, branches off from an inhibin purification procedure. It is expected that FRP, as the desired product, may be isolated using more direct procedures with fewer purification steps.

Various features are emphasized in the following claims.

## Claims

1. A follicle-stimulating hormone-releasing protein (FRP) in substantially pure form, said protein characterized in that it stimulates the secretion and biosynthesis of FSH from cultured anterior pituitary cells but does not stimulate the release of LH, the action of said protein not being blocked by known GnRH antagonists which interact with GnRH receptors on such pituitary cells, said protein having a molecular weight of 28,000 daltons as determined by SDS-PAGE under non-reducing conditions.

2. The FRP of Claim 1 further characterized in that, in an analytical reverse-phase high-performance liquid chromatography (RP-HPLC), with a stationary phase of Vydac $C_8$ ($C_8$-coated particulate silica), a 0.46 x 25 cm. column, and a mobile phase which consists of buffer A, namely 0.1% trifluoroacetic acid in water, and buffer B, namely 20% buffer A and 80% acetonitrile, when the protein-containing specimen is loaded with the mobile phase initially at a flow rate of 1.2 ml/min. and constituting 20% buffer B and with the flow rate thereafter being reduced to 0.7 ml. per minute and a straight line mobile phase gradient being employed to achieve 45% buffer B in 30 minutes, the RP-HPLC being carried out at 40°C, said FRP protein elutes as a sharp band 39 minutes after said starting time of said gradient.

3. The FRP of Claim 1 isolated from mammalian follicular fluid.

4. The FRP of Claim 1 isolated from porcine follicular fluid.

5. The FRP of Claim 1 having an $EC_{50}$ of 0.5 ng/ml.

6. Substantially pure dimers of vertebrate inhibin β subunits or biologically active fragments thereof for use to cause the release of FSH in a vertebrate animal.

7. Substantially pure dimers according to Claim 6 where the dimer is a homodimer of inhibin $\beta_A$ subunits.

8. Substantially pure dimers according to Claim 6 where the dimers are homodimer of inhibin $\beta_B$ subunits.

9. Substantially pure dimers according to Claim 6 where the dimers is a homodimer of inhibin $\beta_B$ and inhibin $\beta_B$ subunits.

10. Substantially pure dimers according to Claim 6 of porcine inhibin $\beta_A$ subunits.

11. Substantially pure dimers according to Claim 6 where each subunit has a formula selected from the formulae: (a) H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH and (b) H-Gly-Leu-Glu-Cys-Asp-Gly-Arg-Thr-Asn-Leu-Cys-Cys-Arg-Gln-Gln-Phe-Phe-Ile-Asp-Phe-Arg-Leu-Ile-Gly-Trp-Ser-Asp-Trp-Ile-Ile-Ala-Pro-Thr-Gly-Tyr-Tyr-Gly-Asn-Tyr-Cys-Glu-Gly-Ser-Cys-Pro-Ala-Tyr-Leu-Ala-Gly-Val-Pro-Gly-Ser-Ala-Ser-Ser-Phe-His-Thr-Ala-Val-Val-Asn-Gln-Tyr-Arg-Met-Arg-Gly-Leu-Asn-Pro-Gly-Thr-Val-Asn-Ser-Cys-Cys-Ile-Pro-Thr-Lys-Leu-Ser-Thr-Met-Ser-Met-Leu-Tyr-Phe-Asp-Asp-Glu-Tyr-Asn-Ile-Val-Lys-Arg-Asp-Val-Pro-Asn-Met-Ile-Val-Glu-Glu-Cys-Gly-Cys-Ala-OH.

12. Substantially pure dimers according to Claim 6 of two 116-residue peptides, interconnected by disulfide bonding, each having the formula: H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH.

## Patentansprüche

1. Follikel-stimulierendes Hormonfreisetzungsprotein (FRP) in im wesentlichen reiner Form, welches Protein dadurch gekennzeichnet ist, daß es die Sekretion und Biosynthese von FSH, welches aus vorderen Hypophysezellen kultiviert wurde, stimuliert, jedoch die Freisetzung von LH nicht stimuliert, wobei die Wirkung des Proteins durch bekannte GnRH-Antagonisten, welche mit GnRH-Rezeptoren auf derartigen Hypophysezellen wechselwirken, nicht blockiert wird und daß das Protein ein Molekulargewicht von 28 000 Dalton aufweist, welches durch SDS-PAGE unter nicht-reduzierenden Bedingungen bestimmt wurde.

2. FRP nach Anspruch 1, dadurch gekennzeichnet, daß in einer analytischen Umkehrphasen-Hochdruckflüssigkeitschromatographie (RP-HPLC), mit einer stationären Phase aus Vydac $C_8$ ($C_8$ beschichtetes partikelförmiges Siliziumdioxid), einer 0,46x25 cm-Säule und einer mobilen Phase bestehend aus Puffer A, nämlich 0,1% Trifluoressigsäure in Wasser, und Puffer B, nämlich 20% Puffer A und 80% Acetonitril, wobei die Protein enthaltende Phase gemeinsam mit der mobilen Phase anfangs mit einem Fluß von 1,2 ml/min geladen wird, und wobei 20% Puffer B enthalten sind, und wobei danach der Fluß auf 0,7 ml/min gesenkt wird und ein geradliniger Gradient der mobilen Phase angewandt wird, um 45% Puffer B in 30 min zu erreichen, die RP-HPLC bei 40°C durchgeführt wird, wobei das FRP-Protein als scharfe Bande 39 min nach der Startzeit des Gradienten eluliert wird.

3. FRP nach Anspruch 1 aus Säuger Follikel-Fluid isoliert.

4. FRP nach Anspruch 1 aus Schweine Follikel-Fluid isoliert.

5. FRP nach Anspruch 1 mit einem $EC_{50}$ von etwa 0,5 ng/ml.

6. Im wesentlichen reine Dimere von Wirbelinhibin β-Untereinheiten oder biologisch wirksamen Fragmenten davon zur Verwendung, um die Freisetzung von FSH bei einem Wirbeltier zu bewirken.

7. Im wesentlichen reine Dimere nach Anspruch 6, worin das Dimer ein Homodimer von Inhibin $\beta_A$-Untereinheiten ist.

8. Im wesentlichen reine Dimere nach Anspruch 6, worin die Dimer Homodimere von Inhibin $\beta_B$-Untereinheiten sind.

9. Im wesentlichen reine Dimere nach Anspruch 6, worin die Dimer Heterodimere von Inhibin $\beta_A$- und Inhibin $\beta_B$-Untereinheiten sind.

10. Im wesentlichen reine Dimere nach Anspruch 6, von Schweine Inhibin $\beta_A$-Untereinheiten.

11. Im wesentlichen reine Dimere nach Anspruch 6, worin jede Untereinheiten eine Formel, gewählt aus den Formeln: (a) H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH und (b) H-Gly-Leu-Glu-Cys-Asp-Gly-Arg-Thr-Asn-Leu-Cys-Cys-Arg-Gln-Gln-Phe-Phe-Ile-Asp-Phe-Arg-Leu-Ile-Gly-Trp-Ser-Asp-Trp-Ile-Ile-Ala-Pro-Thr-Gly-Tyr-Tyr-Gly-Asn-Tyr-Cys-Glu-Gly-Ser-Cys-Pro-Ala-Tyr-Leu-Ala-Gly-Val-Pro-Gly-Ser-Ala-Ser-Ser-Phe-His-Thr-Ala-Val-Val-Asn-Gln-Tyr-Arg-Met-Arg-Gly-Leu-Asn-Pro-Gly-Thr-Val-Asn-Ser-Cys-Cys-Ile-Pro-Thr-Lys-Leu-Ser-Thr-Met-Ser-Met-Leu-Tyr-Phe-Asp-Asp-Glu-Tyr-Asn-Ile-Val-Lys-Arg-Asp-Val-Pro-Asn-Met-Ile-Val-Glu-Glu-Cys-Gly-Cys-Ala-OH aufweist.

12. Im wesentlichen reine Dimere nach Anspruch 6, bestehend aus zwei 116-Reste Peptiden, welche durch Disulfidbindungen ver-bunden sind, welche jeweils die Formel:
H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH aufweisen.

## Revendications

1. Protéine libérant l'hormone sécrétant la folliculostimuline (FRP), sous une forme substantiellement pure, ladite protéine étant caractérisée en ce qu'elle stimule la sécrétion et la biosynthèse de FSH de cellules antéhypophysaires en culture, mais ne stimule pas la libération de la LH, l'action de ladite protéine n'étant pas bloquée par des antagonistes de GnRH connus, qui agissent mutuellement avec des récepteurs de GnRH sur de telles cellules hypophysaires, ladite protéine ayant un poids moléculaire de 28.000 daltons, tel qu'il est déterminé par électrophorèse sur gel de dodécylsulphate de sodium et d'acrylamide dans des conditions de non-réduction.

2. FRP selon la revendication 1, caractérisé en ce que dans une chromatographie liquide à haute performance à phase inverse (RP-HPLC), avec une phase fixe de silice particulaire recouverte de Vydac $C_8$, une colonne de 0,46 x 25 cm et une phase mobile composée d'un tampon A, à savoir 0,1 % d'acide trifluoroacétique dans de l'eau, et d'un tampon B, à savoir 20 % de tampon A et 80 % d'acétonitrile, quand l'échantillon contenant la protéine est chargé avec la phase mobile, à une vitesse d'écoulement initiale de 1,2 ml/minute et formant 20 % de tampon B, la vitesse d'écoulement étant réduite ensuite à 0,7 ml par minute, et un gradient linéaire à phase mobile étant utilisé pour obtenir 45 % de tampon B en 30 minutes, la RP-HPLC étant réalisée à 40°C, ladite protéine FRP est éluée sous la forme d'une bande distincte, 39 minutes après le temps de démarrage dudit gradient.

3. FRP selon la revendication 1, isolée dans du liquide folliculaire mammifère.

4. FRP selon la revendication 1, isolée dans du liquide folliculaire porcin.

5. FRP selon la revendication 1, ayant une valeur $EC_{50}$ de 0,5 ng/ml.

6. Dimères substantiellement purs formés de sous-unités β d'inhibine de vertébrés, ou de fragments biologiquement actifs de celles-ci, destinés à être utilisés pour provoquer la libération de FSH chez un animal vertébré.

7. Dimères substantiellement purs selon la revendication 6, où le dimère est un homodimère de sous-unités $\beta_A$ d'inhibine.

8. Dimères substantiellement purs selon la revendication 6, où les dimères sont des homodimères de sous-unités $\beta_B$ d'inhibine.

9. Dimères substantiellement purs selon la revendication 6, où les dimères sont des hétérodimères de sous-unités $\beta_A$ d'inhibine et de sous-unités $\beta_B$ d'inhibine.

10. Dimères substantiellement purs selon la revendication 6, formés de sous-unités $\beta_A$ d'inhibine porcine.

11. Dimères substantiellement purs selon la revendication 6, dans lesquels chaque sous-unités a une formule choisie parmi les formules : (a) H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH et (b) H-Gly-Leu-Glu-Cys-Asp-Gly-Arg-Thr-Asn-Leu-Cys-Cys-Arg-Gln-Gln-Phe-Phe-Ile-Asp-Phe-Arg-Leu-Ile-Gly-Trp-Ser-Asp-Trp-Ile-Ile-Ala-Pro-Thr-Gly-Tyr-Tyr-Gly-Asn-Tyr-Cys-Glu-Gly-Ser-Cys-Pro-Ala-Tyr-Leu-Ala-Gly-Val-Pro-Gly-Ser-Ala-Ser-Ser-Phe-His-Thr-Ala-Val-Val-Asn-Gln-Tyr-Arg-Met-Arg-Gly-Leu-Asn-Pro-Gly-Thr-Val-Asn-Ser-Cys-Cys-Ile-Pro-Thr-Lys-Leu-Ser-Thr-Met-Ser-Met-Leu-Tyr-Phe-Asp-Asp-Glu-Tyr-Asn-Ile-Val-Lys-Arg-Asp-Val-Pro-Asn-Met-Ile-Val-Glu-Glu-Cys-Gly-Cys-Ala-OH.

12. Dimères substantiellement purs selon la revendication 6, formés de deux peptides à 116 résidus, reliés entre eux par une liaison disulfure, chacun d'eux répondant à la formule :
H-Gly-Leu-Glu-Cys-Asp-Gly-Lys-Val-Asn-Ile-Cys-Cys-Lys-Lys-Gln-Phe-Phe-Val-Ser-Phe-Lys-Asp-Ile-Gly-Trp-Asn-Asp-Trp-Ile-Ile-Ala-Pro-Ser-Gly-Tyr-His-Ala-Asn-Tyr-Cys-Glu-Gly-Glu-Cys-Pro-Ser-His-Ile-Ala-Gly-Thr-Ser-Gly-Ser-Ser-Leu-Ser-Phe-His-Ser-Thr-Val-Ile-Asn-His-Tyr-Arg-Met-Arg-Gly-His-Ser-Pro-Phe-Ala-Asn-Leu-Lys-Ser-Cys-Cys-Val-Pro-Thr-Lys-Leu-Arg-Pro-Met-Ser-Met-Leu-Tyr-Tyr-Asp-Asp-Gly-Gln-Asn-Ile-Ile-Lys-Lys-Asp-Ile-Gln-Asn-Met-Ile-Val-Glu-Glu-Cys-Gly-Cys-Ser-OH.

FIG. I

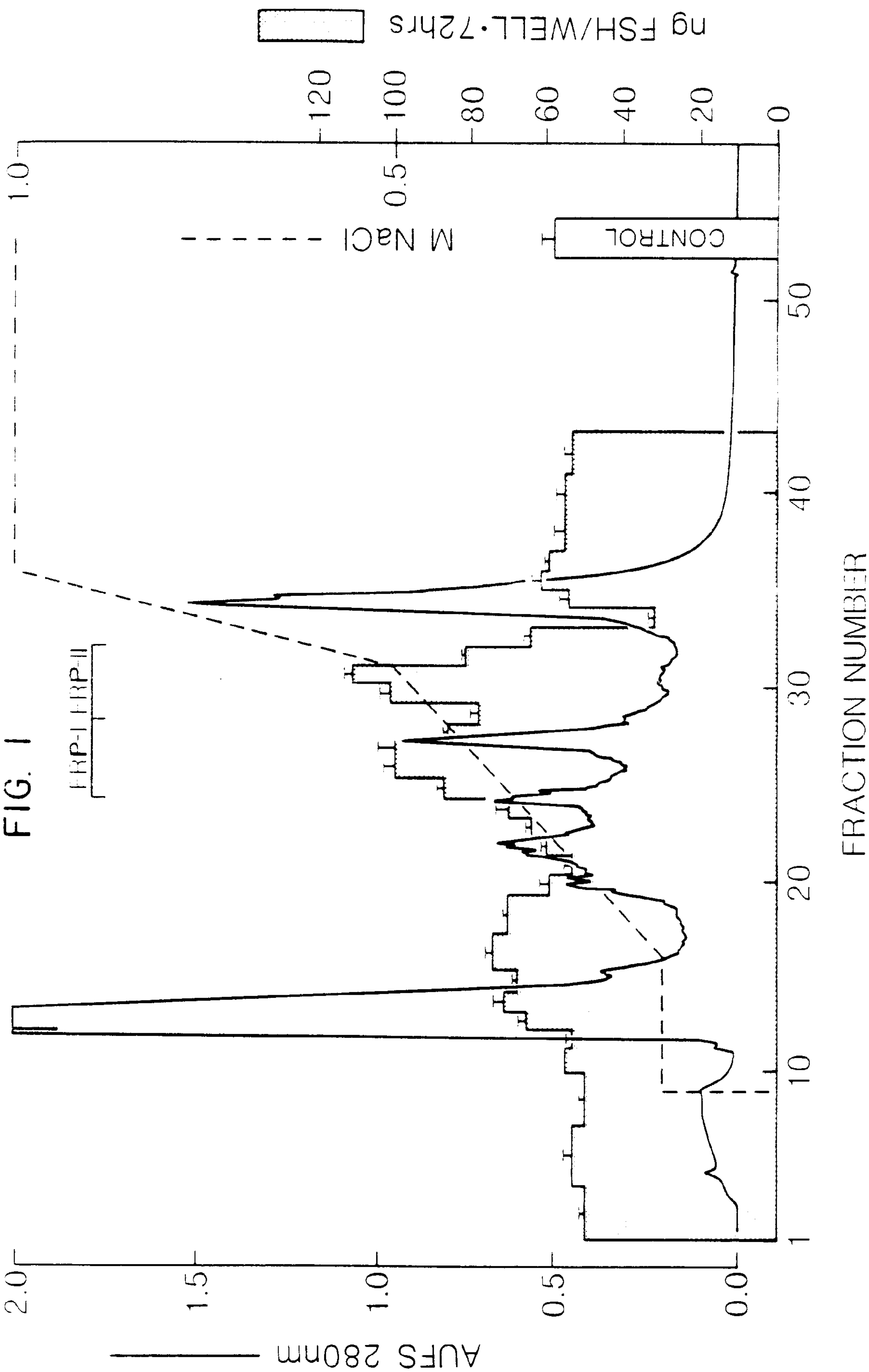
AUFS 280nm
ng FSH/WELL·72hrs
M NaCl
CONTROL
FRP-I
FRP-II
FRACTION NUMBER
2.0
1.5
1.0
0.5
0.0
120
100
80
60
40
20
0
1
10
20
30
40
50

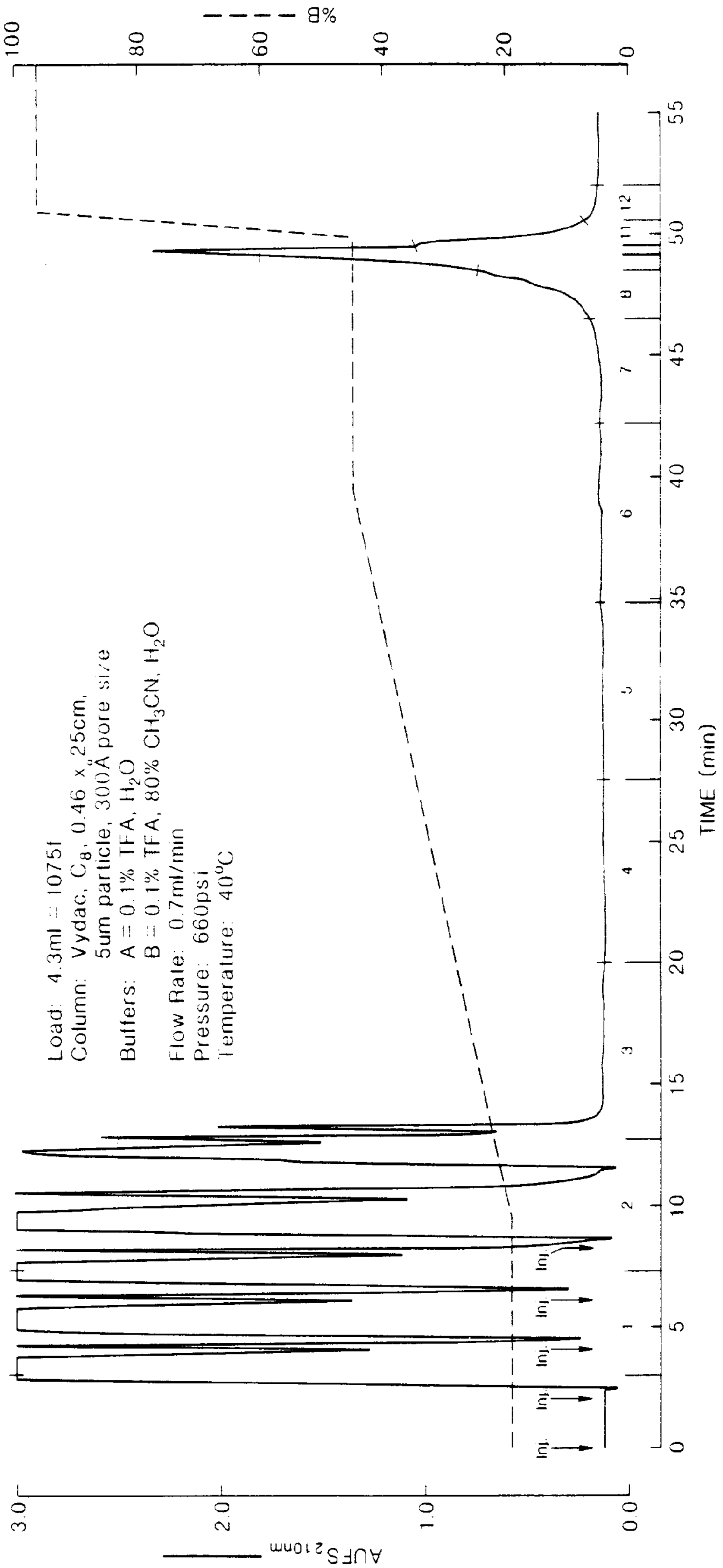
Load: 4.3ml = 1075f
Column: Vydac, $C_8$, 0.46 x 25cm, 5um particle, 300Å pore size
Buffers: A = 0.1% TFA, $H_2O$
B = 0.1% TFA, 80% $CH_3CN$, $H_2O$
Flow Rate: 0.7ml/min
Pressure: 660psi
Temperature: 40°C
AUFS$_{210nm}$
%B
TIME (min)
Inj.

FIG. 2